(19)

**Europäisches Patentamt**

**European Patent Office**

**Office européen des brevets**

(11) **EP 4 745 983 A1**

(12) **EUROPEAN PATENT APPLICATION**

(43) Date of publication:
20.05.2026 Bulletin 2026/21

(21) Application number: 25212327.8

(22) Date of filing: 30.10.2025

(51) International Patent Classification (IPC):
*G16H 30/40* (2018.01)      *G16H 40/40* (2018.01)

(52) Cooperative Patent Classification (CPC):
**G16H 30/40; G16H 40/40;** G16H 40/60;
G16H 40/63

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **13.11.2024 EP 24212547**

(71) Applicant: **Stryker European Operations Limited**
**Carrigtwohill, Co. Cork T45 HX08 (IE)**

(72) Inventors:
• FISCHER, Lars
79110 Freiburg (DE)
• KHORWAL, Renu
110063 New Delhi (IN)
• HORNECKER, Patrick
79356 Eichstetten (DE)
• Uhl, Annika
79106 Freiburg (DE)

(74) Representative: **Schott, Jakob Valentin**
**Wuesthoff & Wuesthoff**
**Patentanwälte und Rechtsanwalt PartG mbB**
**Schweigerstraße 2**
**81541 München (DE)**

(54) **TECHNIQUE FOR INFORMING A USER ON A TIME ASSOCIATED WITH RECEIVING MEDICAL PATIENT SCAN DATA**

(57) Disclosed is a method for informing a user of a surgical navigation system on a time required to receive medical patient scan data from an image providing unit. A total number of images comprised in medical patient scan data to be received by the surgical navigation system from an image providing unit is determined. Based on the total number of images, the total time required to receive, by the surgical navigation system, the medical patient scan data from the image providing unit is determined. An indication of the determined total time or information derived from the determined total time is triggered to be output. A surgical navigation system, a medical system, a computer program and a carrier are also disclosed.

Fig. 2

EP 4 745 983 A1

## Description

### Technical Field

The present disclosure generally relates to a method for informing a user of a surgical navigation system on a time associated with receiving medical patient scan data from an image providing unit. A surgical navigation system, a medical system, a computer program and a carrier are also provided.

### Background

Medical patient scan data is frequently used for surgical planning, surgical navigation and validation. Such medical patient scan data may comprise x-ray images or magnetic resonance, MR, images. A typical type of patient scan is the so-called computed tomography, CT, scan.

In recent years, the resolution of image providing units that can acquire such patient scans have increased. Therefore, the overall file size of the medical patient scan data has also increased. In turn, the time required for transmitting the medical patient scan data from the image providing unit to a surgical navigation system, where it may be used for planning, navigation or validation, has also increased compared with lower-size medical patient scan data containing, for example, a low-resolution CT patient scan. The time required for transmitting a high-resolution patient scan from a modern image providing unit to a surgical navigation system can amount to dozens of minutes and may be hard to predict by surgical staff.

Surgical staff may abort the data transfer from the image providing unit to the surgical navigation system prematurely if they do not want to wait any longer for the transfer to be completed and/or if they assume that the transfer has been completed. Also, in the absence of any information regarding the time associated with receiving the medical patient scan data from the image providing unit, subsequent procedural steps such as surgery preparation, patient (re-) positioning, instrument preparation and the like can hardly be planned in an efficient manner. Still further, without such information, a surgeon may conduct subsequent patient scans using the same scan settings without considering the time associated with receiving the medical patient scan data.

### Summary

There is a need for a technique that solves one or more of the aforementioned or other problems.

According to a first aspect, a computer-implemented method for informing a user of a surgical navigation system on a time associated with receiving medical patient scan data from an image providing unit is provided. The method is performed by at least one processor of the surgical navigation system. The method comprises determining a total number of images comprised in medical patient scan data to be received by the surgical navigation system from the image providing unit. The method comprises determining, based on the total number of images, the total time required to receive, by the surgical navigation system, the medical patient scan data from the image providing unit. The method comprises triggering output of an indication of the determined total time or information derived from the determined total time.

The method may not comprise a surgical step. The user of the surgical navigation system may be clinical staff, for example a surgeon or a radiologist. The time may be associated with receiving the medical patient scan data from the image providing unit by indicating or corresponding to a (e.g., total) time that is required for receiving the medical patient scan data from the image providing unit. The surgical navigation system may comprise a memory (e.g., the carrier of the fifth aspect mentioned below) storing instructions that, when executed by the at least one processor, caused the at least one processor to perform the method of the first aspect. The medical patient scan data may comprise or consist of one or more medical patient images, for example of a single or multiple patient scans. The medical patient scan data in one example only contains images of a single patient scan. The medical patient scan data may correspond to a dataset of a CT patient scan. The image providing unit may be configured to acquire one or more medical (e.g., x-ray and/or MR) images of a patient. The image providing unit may be configured to conduct a patient scan to generate the medical patient scan data. The indication of the determined total time or information derived from the determined total time may be triggered to be output by at least one output unit of the surgical navigation system. Said indication may be or comprise a visual indication, an auditory indication and/or a haptic indication. Said indication may be triggered to be displayed by a display device (e.g., part of the surgical navigation system or separate therefrom).

For example, all images comprised in the medical patient scan data are associated with a same medical scan (e.g., a same medical image acquisition scan) of a same patient, which may also be referred to as patient scan herein. In this case, all images may have been acquired (e.g., by the image providing unit) when said same medical scan was performed. The medical scan may also be referred to as patient scan herein.

The total number of images may be determined based on a scan path of the medical scan. In one example, the scan path is predefined and/or known to the surgical navigation system (e.g., indicated by scan path information obtained from an external entity). In another example, the scan path is determined by the surgical navigation system.

For example, the scan path defines a relative movement between the patient and an image capturing volume that occurred during the medical scan. The scan path may be a curve (e.g., a helix) or a straight line. The image capturing volume may correspond to a spatial volume that the image providing unit is capable of imaging at a given point in time. In one variant, the scan path defines a movement of the image capturing volume with respect to the stationary patient. In another variant, the scan path defines a movement of a patient and/or a patient couch supporting the patient, relative to the stationary image capturing volume. A combination of both variants is also possible.

The total number of images may be determined based on a (e.g., spatial) length of the scan path. In one example, the length of the scan path is predefined and/or known to the surgical navigation system (e.g., indicated by length information obtained from an external entity). In another example, the length of the scan path is determined by the surgical navigation system.

The length of the scan path may be determined based on a spatial start position and a spatial end position of the image capturing volume relative to the patient. For example, the scan path extends (e.g., continuously) between the spatial start position and the spatial end position. In one example, the spatial start position and/or the spatial end position is predefined and/or known to the surgical navigation system (e.g., indicated by position information obtained from an external entity). In another example, the spatial start position and/or the spatial end position is determined by the surgical navigation system.

Alternatively, or in addition, the length of the scan path may be determined based on a movement time of the image capturing volume relative to the patient (e.g., along the scan path) and/or based on a movement speed of the image capturing volume relative to the patient (e.g., along the scan path). In one example, the movement time and/or the movement speed is predefined and/or known to the surgical navigation system (e.g., indicated by movement information obtained from an external entity). In another example, the movement time and/or the movement speed is determined by the surgical navigation system.

For example, the total number of images is determined based on an amount of static (e.g., periodical) positions along the scan path (e.g., between the patient and the image capturing volume). Each of these static positions may have been present (e.g., at a different time) when the medical scan was conducted. Each static position may correspond to a position at which a medical image (e.g., comprised in the medical patient scan data) was acquired. The static positions may also be referred to as imaging positions herein. The spatial start position and/or the spatial end position may also be an imaging position. In one example, the (e.g., amount of) static positions is predefined and/or known to the surgical navigation system (e.g., indicated by imaging position information obtained from an external entity). In another example, the (e.g., amount of) static positions is determined by the surgical navigation system. The amount of static positions may be determined by dividing the length of the scan path by a slice thickness. Each static position may be associated with a predefined number of images that may be specific for a type of the medical scan, a type of the imaging unit or and/ other parameters known to the surgical navigation system.

The total number of images may be determined based on a type of the medical scan (e.g., a type of medical images comprised in the medical patient scan data) and/or based on a slice thickness of the medical scan. Examples of different types of the medical scan are i) an axial (e.g., CT) scan and ii) a helical (e.g., CT) scan. The slice thickness may correspond to a distance between adjacent static (e.g., imaging) positions. The slice thickness may correspond to a spatial offset between a first pose of the imaging volume relative to the patient and a (e.g., adjacent or immediately following) second pose of the imaging volume relative to the patient. In one example, the type of the medical scan and/or the slice thickness is predefined and/or known to the surgical navigation system (e.g., indicated by scan information obtained from an external entity). In another example, the type of the medical scan and/or the slice thickness is determined by the surgical navigation system.

The total number of images may be determined as a sum of the predefined numbers of images. If this number is equal for each static position, the total number of images may be determined by multiplying the predefined number of images with the amount of static positions along the scan path. The amount of static positions may be determined based on the length of the scan path and the slice thickness, for example by dividing said length by said slice thickness.

For example, at least one of {i} the scan path, {ii} the length of the scan path, {iii} the spatial start position, {iv} the spatial end position, {v} the movement time, {vi} the movement speed, {vii} the static positions, {viii} the type of the medical scan, and {ix} the slice thickness of the medical scan are captured when the medical scan is conducted. The method may comprise capturing the at least one of {i} to {ix}, for example when the medical scan is conducted.

The capturing may be based on a message received from the image providing unit and/or based on tracking data received from a tracking unit. The capturing may be based on one or more of the scan path information, the length information, the position information, the movement information, the imaging position information and the scan information. One or more of said information may be contained in the message received from the image providing unit and/or in the tracking data received from the tracking unit. The method may comprise deriving one or more of the scan path information, the length information, the position information, the movement information, the imaging position information and the scan information from the message received from the image providing unit and/or from the tracking data received from the tracking unit. Accordingly, the external entity mentioned herein above may correspond to the image providing unit and/or the tracking unit.

The tracking data may be indicative of one or more relative poses between the image providing unit and the patient or patient couch. Each of said poses may be associated with a same patient scan. The tracking data may be indicative of one or more first poses of the image providing unit relative to a camera of the tracking unit and indicative of one or more second poses of the patient or patient couch relative to the camera. The tracking unit may be configured as an optical tracking unit comprising at least one camera configured to acquire one or more images depicting (e.g., an optical tracker attached to) the image providing unit and (e.g., another optical tracker attached to) the patient or patient couch (e.g., at different points in time).

For example, the total time is determined based on an estimated data transfer rate between the image providing unit and the surgical navigation system. In one example, the estimated data transfer rate is predefined and/or known to the surgical navigation system (e.g., indicated by connection information obtained from an external entity). In another example, the estimated data transfer rate is determined by the surgical navigation system, for example based on one or more previous messages (e.g., ping messages, connection establishment messages, or datacontaining messages) transmitted between the image providing unit and the surgical navigation system. For example, the estimated data transfer rate may be determined using a round-trip-time, RTT, technique.

An actual data transfer rate between the image providing unit and the surgical navigation system may be determined (e.g., while receiving the medical patient scan data from the image providing unit). The actual data transfer rate may be used to update the determined total time and/or the information derived from the determined total time. The indication triggered to be output may then also be updated accordingly.

For example, the information derived from the determined total time comprises or is a remaining time required for receiving (e.g., a remainder of) the medical patient scan data from the image providing unit. For example, the information derived from the determined total time comprises or is a proportion of i) lapsed time for receiving the medical patient scan data from the image providing unit and ii) the total time. For example, the information derived from the determined total time comprises or is a proportion of iii) remaining time for receiving (e.g., a reminder of) the medical patient scan data from the image providing unit and iv) the total time.

In one example, the method further comprises triggering output of an indication of the determined total number of images or of information derived from the determined total number of images. The indication of the determined total number of images or of information derived from the determined total number of images may be triggered to be output by (e.g., the) at least one output unit of the surgical navigation system. Said indication may be or comprise a visual indication, an auditory indication and/or a haptic indication. Said indication may be triggered to be displayed by a (e.g., the) display device (e.g., part of the surgical navigation system or separate therefrom). For example, the information derived from the determined total number of images comprises or is a remaining number of images of the medical patient scan data that is to be received from the image providing unit. For example, the information derived from the determined total number of images comprises or is a proportion of i) a number of images of the medical patient scan data that was received from the image providing unit and ii) the total number of images. For example, the information derived from the determined total number of images comprises or is a proportion of iii) a number of remaining images of the medical patient scan data that are to be received from the image providing unit and iv) the total number of images.

In one particular variant currently not covered by the claims but falling within the scope of the present disclosure, the method does not need to comprise the step of determining the total time. In this particular variant, the method may also not comprise the step of triggering output of the indication of the determined total time of information derived from the determined total time. In this particular variant, the method may comprise the step of triggering output of an indication of the determined total number of images or of information derived from the determined total number of images.

According to a second aspect, a surgical navigation system is provided. The surgical navigation system comprises at least one processor that is configured to perform the method according to the first aspect. The surgical navigation system may comprise a memory (e.g., the carrier of the fifth aspect mentioned below) storing instructions that, when executed by the at least one processor, cause the at least one processor to perform the method of the first aspect. The surgical navigation system may comprise an interface configured to be communicatively coupled to the image providing unit. The surgical navigation system may comprise the at least one output unit, for example a speaker, a display or a haptic user interface. The at least one output unit may be configured to output the indication(s) that is (are) triggered to be output.

According to a third aspect, a medical system is provided. The medical system comprises the surgical navigation system of the second aspect. The medical system further comprises the image providing unit. The image providing unit may comprise a detector module configured to acquire the images comprised in (e.g., the) medical patient scan data. The image providing unit may be configured as a CT scanner or an MR scanner. The image providing unit may be configured as an O-ring imaging apparatus or as a C-arm imaging apparatus. The images (e.g., acquired by the image providing unit) may comprise x-ray images and/or magnetic resonance images. The image providing unit may comprise an interface configured to be communicatively coupled to the surgical navigation system. For example, the image providing unit is connected to the surgical navigation system via a local network connection. Alternatively, or in addition, the image providing unit and the surgical navigation system may be (e.g., configured to be) arranged within a same hospital. The medical system may further comprise the patient couch configured to support the patient at least during acquisition of a (e.g., the) medical scan. The medical system may further comprise the tracking unit. The tracking unit may be configured as an optical tracking unit comprising at least one camera configured to acquire one or more images depicting (e.g., an optical tracker attached to) the image providing unit and (e.g., another optical tracker attached to) the patient or patient couch (e.g., at different points in time). The tracking unit may be communicatively coupled to the surgical navigation system. The medical system may further comprise a first (e.g., optical) tracker that is attached to the imaging unit and a second (e.g., optical) tracker that is attached to the patient or the patient couch. The tracking unit may be configured to localize these trackers (e.g., relative to one another or relative to a common reference such as a camera of the tracking unit).

According to a fourth aspect, a computer program is provided. The computer program comprises instructions which, when executed by at least one processor of a surgical navigation system, cause the at least one processor to perform the method according to the first aspect. The computer program may be carried by at least one carrier such as memory, a non-transitory computer-readable storage medium or a data stream.

According to a fifth aspect, a carrier is provided. The carrier carries (e.g., stores) the computer program of the fourth aspect. The carrier may be a memory, a non-transitory computer storage medium or a data stream.

**Brief Description of the Drawings**

Further details, advantages and aspects of the present disclosure will become apparent from the following embodiments taken in conjunction with the drawings, wherein:

Fig. 1    shows an exemplary medical system in accordance with the present disclosure; and

Fig. 2    shows a flowchart of an exemplary method in accordance with the present disclosure.

**Detailed Description**

In the following description, exemplary embodiments will be explained with reference to the drawings. Unless indicated otherwise, the reference signs used in the following denote the same or similar structural or functional features.

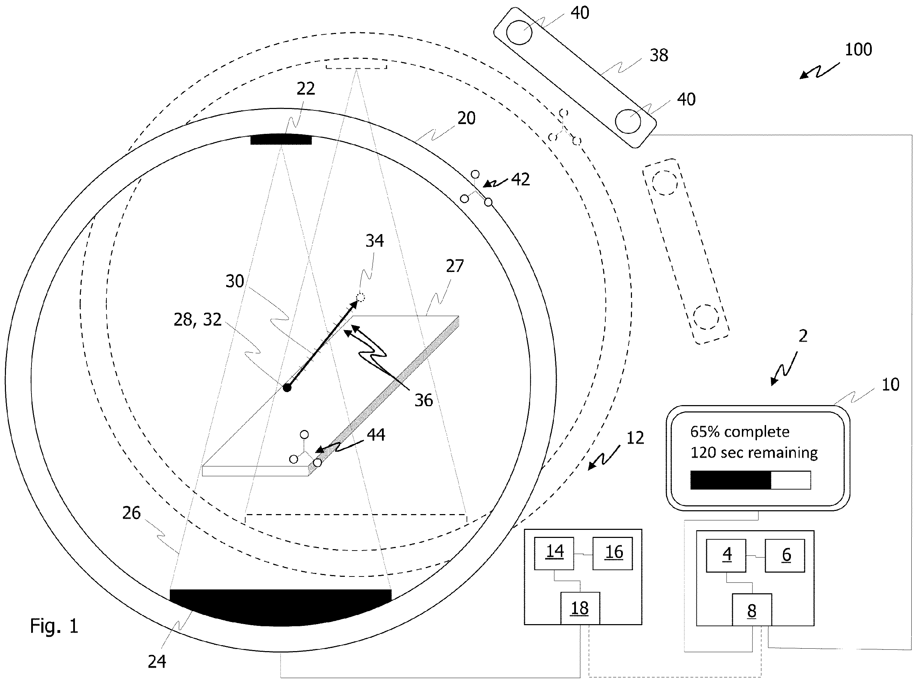

Fig. 1 shows an exemplary medical system 100 in accordance with the present disclosure.

The medical system 100 comprises a surgical navigation system 2 including a processor 4 coupled to a memory 6 and an interface 8. The surgical navigation system 2 comprises a display 10 coupled to the processor 4 via the interface 8. The memory 6 stores instructions which, when executed by the processor 4, cause the processor 4 to perform the method 200 described herein below.

The medical system 100 further comprises an image providing unit 12 including a processor 14 coupled to a memory 16 and an interface 18. The interface 18 is communicatively coupled to the interface 8. The image providing unit 12 is configured to conduct patient scans to thereby generate medical patient scan data. This data may then be transferred to the surgical navigation system 2 via the interfaces 18, 8, for example across a local network connection.

The image providing unit 12 in the illustrated example is configured as an O-ring CT scanner comprising an O-shaped gantry 20 carrying an irradiation source 22 and an irradiation detector 24 facing one another. The image providing unit 12 is capable of capturing images that depict an image capturing volume 26 lying between the source 22 and the detector 24. The gantry 20 is translationally movable relative to a patient couch 27 of the medical system 100. The gantry 20 is further able to rotate around the patient couch 27 around a center of rotation 28, also referred to as isocenter. A patient lies on the patient couch 27 when the patient scan is conducted.

Fig. 1 indicates a scan path 30 along which the volume 26 and, thus, also the isocenter 28 move when the image providing unit 12 conducts a patient scan. The initial pose of the gantry 20 is indicated in solid lines, whereas its pose at the end of the patient scan is indicated in dashed lines. The scan path 30 extends linearly from a spatial start position 32 to a spatial end position 34. A plurality of equally-spaced imaging positions 36 lie along the scan path 30. At each of these imaging positions 36, the image providing unit 12 may acquire one or more medical images using the detector 24, the one or more images forming part of the same patient scan. One may say that the distance between adjacent imaging positions 36 corresponds to a slice thickness of the patient scan.

In case of an axial patient scan, the gantry 20 may stop at each of these static imaging positions 36 and acquire multiple images with the gantry 20 having different rotational poses. The gantry 20 may then move over to the next position 36 and repeat the image acquisition process there.

In case of a helical patient scan, the isocenter 28 may continuously move along the scan path while the gantry 20 rotates therearound. Also in this case, one or more medical images may be acquired each time the isocenter 28 is at one of the imaging positions 36.

The medical system 100 further comprises a tracking unit 38 comprising a stereocamera 40, a first optical tracker 42 attached to the gantry 20 and a second optical tracker 44 attached to the patient couch 27. In another variant, the tracker 44 is attached to a patient lying on the patient couch 27 while a medical scan is conducted by the image providing unit. The tracking unit 38 captures images with the stereocamera 40 and forwards these images to the surgical navigation system 2 via the interface 8, and the system 2 then localizes the three-dimensional poses of the trackers 42, 44 in the images. Alternatively, these poses may be determined by the tracking unit 38 itself, and then be transmitted to the surgical navigation system 2. The position of the isocenter 28 relative to the first optical tracker 42 is predefined (e.g., calibrated in advance). Thus, the surgical navigation system 2 can derive the start position 32 and the end position 34 from tracking data obtained from the tracking unit 38. In case of an axial scan, the imaging positions 36 can also be derived from the tracking data. The tracking data also allows the surgical navigation system 2 to derive a movement time of the gantry 20 during the patient scan, a movement speed of the gantry 20 during the patient scan, the length and/or shape of the scan path 30, and other scan-related information. In some variants, the pose of the tracking unit 38 may change over time, as indicated in Fig. 1 in dashed lines. By building on the relative poses between the trackers 42, 44, such a pose change does not affect the tracking results and, in turn, the scan-related information obtained via the tracking unit 38.

It should be noted, that it is alternatively or additionally possible for the image providing unit 12 to feed this and other scan-related information to the surgical navigation system 2, for example as part of a (e.g., Digital Imaging and Communications in Medicine, DICOM) header of a medical image comprised in the medical patient scan data. Furthermore, it is possible to use a tracking unit 38 that directly tracks the relative poses of the image providing unit and the patient or patient couch, for example using a machine vision algorithm that localizes these entities in a (e.g., color and/or stereo) image. In this case, the trackers 42, 44 may be omitted. Combinations of these approaches are also possible. For example, a part of scan-related information may be obtained via the tracking unit 38 tracking the trackers 42, 44, another part may be obtained from the image providing unit 12 and a still further part may optionally be obtained via the tracking unit 38 directly tracking the gantry 20 relative to the patient or patient couch 27.

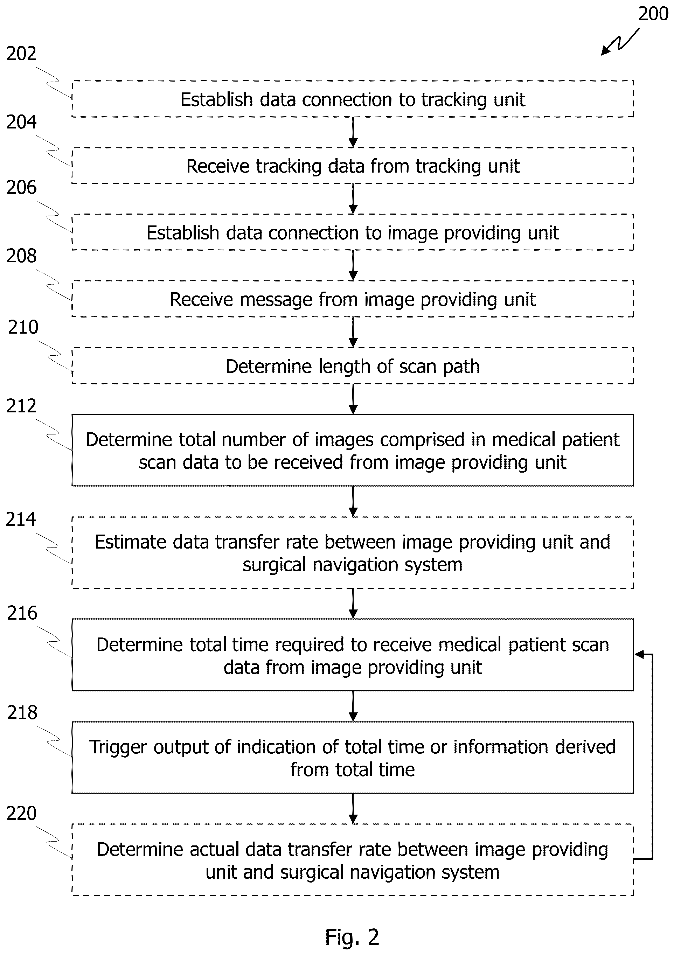

Fig. 2 shows a flowchart of an exemplary method 200 in accordance with the present disclosure. Optional features are indicated in dashed boxes. The method may be performed by the at least one processor 4 of the surgical navigation system 2.

At 202, a data connection is established between the surgical navigation system 2 and the tracking unit 38 via the interface 8.

At 204, tracking data is received from the tracking unit 38. As described above, the tracking data may be indicative of one or more relative poses between the image providing unit 102, in particular the gantry 20 or the tracker 42 attached thereto, and the patient or patient couch 27, in particular the tracker 44. The tracking data may be indicative of said one or more poses that were present during a patient scan. The tracking data may be received and configured to indicate said one or more poses "live", i.e., during the patient scan. The surgical navigation system 2 may derive some or all scan information from the tracking data, for example one or more of {i} the scan path 30, {ii} the length of the scan path 30, {iii} the spatial start position 32, {iv} the spatial end position 34, {v} the movement time, {vi} the movement speed, {vii} the static positions 36 and {ix} the slice thickness of the medical scan.

At 206, a data connection is established between the surgical navigation system 2 and the image providing unit 12 via the interfaces 8, 18. This may involve testing or determining a data transfer rate between the system 2 and the unit 12 or vice versa, for example using one or more ping messages and/or a RTT procedure.

At 208, one or more messages are received by the surgical navigation system 2 from the image providing unit 12. The message(s) may indicate some or all scan information mentioned above and/or other scan information, for example {viii} a type of the medical scan, {ix} the slice thickness and/or {x} a model of the image providing unit 12.

Thus, at 210, the length of the scan path 30 is determined based on the tracking data and/or the one or more messages received from the image providing unit 12. The length of the scan path 30 may be determined as the distance between the start position 32 and the end position 34 in case of a linear scan path 30. In case of a non-linear scan path 30, the length of the scan path 30 may be determined by calculating its overall spatial length (e.g., across multiple spatial dimensions). The length of the scan path may be determined based on a movement and/or a plurality of relative poses of the gantry 20 relative to the patient or patient couch 27. The length may be determined by multiplying a relative movement speed between these two entities present during the patient scan with a total movement time thereof during said patient scan. The length of the scan path may not be derivable from medical patient scan data.

At 212, a total number of images comprised in medical patient scan data to be received by the surgical navigation system 2 from the image providing unit 12 is determined. The total number of images may be determined based on the length of the scan path 30 as determined at 210. The total number of images may be determined based on the number of static positions 36. This number may be determined by dividing the length of the scan path 30 by the slice thickness. In case the type of the patient scan is associated with more than one image acquisition (e.g., a predefined number of acquired images) at each of the static positions 36 (e.g., in case of an axial CT scan), the number of static positions may be multiplied by said predefined number of acquired images to determine the total number of images of the patient scan, that are part of the medical patient scan data.

At 214, a data transfer rate between the unit 12 and the system 2 via the interfaces 8, 18 is estimated. As described above, this procedure may also be performed in advance, for example at 206.

At 216, a total time required for the surgical navigation system 2 to receive the medical patient scan data from the image providing unit 12 is determined. The total time may be determined based on the total number of images as determined at 212 and based on the estimated data transfer rate. The total time may be estimated based on a predefined, indicated or predicted average image size of the medical patient images contained in the medical patient image data. For example, said size may be associated with the type of the patient scan and/or the type or model of the image providing unit. Said size may be derived from a first medical image of the medical patient scan data that is received by the surgical navigation system. Said size may be indicated by one or the messages at 208 and/or as part of a DICOM header of one of the medical images of the medical patient scan data.

At any point before 218, a transfer of the medical patient scan data from the image providing unit 12 to the surgical navigation system 2 may be triggered, for example by a user or automatically upon the image providing unit 12 finishing the patient scan. Then, at 218, an indication of the total time or an indication of information derived from the total time is triggered to be output, for example as a visualization on the display 10. The information derived from the total time may comprise one or more of a remaining time, a proportion of remaining and total time, a proportion of remaining and lapsed time and a predicted time point at which the data transfer will be completed. As shown in the example of Fig. 1, the total time or the information derived therefrom may be displayed as text, numbers or a progress bar.

At 220, an actual data transfer rate between the image providing unit 12 and the surgical navigation system 2 is determined, for example based on the ongoing transfer of the medical patient scan data. The actual data transfer rate may then be used to update the total time at 216, which, in turn, may also update the triggered output of 218.

In this manner, the user gains a valuable insight on the progress of the transfer of the medical patient scan data. The indication may even be output to a planning system or one or more clinical machines to configure the same in dependence of said progress (e.g., based on the predicted time point at which the data transfer will be completed), which may make clinical workflows more efficient.

The technique disclosed herein will now be explained in other words.

During navigated surgeries, there may be a need to perform intraoperative imaging with an image providing unit such 12 as a C-arm or a CT scanner. When a user uses an intraoperative CT scanner, there is the risk of having scans that are not fully transferred to the surgical navigation system 2. The present disclosure enables handling this issue by the navigation system 2 determining the length of the scan path 30 and informing the user if it detects a problem (e.g., if the total time exceeds a predefined maximum threshold, for example at 218). Also, if the length of the scan path 30 is known, the navigation system 2 can show the user an estimated time (e.g., at 218) until he might continue as well as a transfer progress.

To be able to register a CT scan, the following pre-conditions should be met:

1. The CT scanner should be trackable, hence a tracker 42 should be attached to it (further on called imaging device tracker).
2. The system 2 should track and store the spatial relation between the tracked CT scanner and a patient or patient couch tracker 44.
3. A pre-calculated calibration matrix, that contains the spatial relationship between the tracked CT scanner 20 and the resulting images should be available and stored by the system 2.
4. The CT scan should be transferred to the navigation system 2 to use it further.

If those elements are known, a registration can be calculated. Once the resulting medical patient scan data is sent to the navigation system, it may be registered automatically and can be used for surgical navigation. The calibration matrix may point towards the isocenter 28 of the CT scanner 12, which is the rotation center of the CT scanner's gantry 20 at the first slice (e.g., at the start position 32). Depending on the exact scanner model, either the patient table 27 is moved through the gantry 20, or the gantry 20 is moved around the patient during scanning.

When transferring the medical patient image scan data containing all medical images of the CT patient scan, there may be a fixed set of images to be transferred for a C-arm volume, so it can be shown to the user how many are there and how many to go. For some CT scanners, the length of the scan path 30 can be individually set when setting up the scan. Therefore, a DICOM volume and number of slices might differ for each scan. If the number of slices were known, the user experience could be improved by showing progress. Even an estimated number of slices to be transferred could help to give some guidance.

To get the number of slices, which may correspond to the number of static positions 36 (e.g., plus one), the system 2 may determine the length of the scan path 30 and the slice distance to find out the number. Multiple approaches are possible to find out the length of the scan path 30. Those might be used individually or combined to compensate when one of the approaches fails in a certain scenario. The slice distance may correspond to the distance of the center between two neighboring slices and may not be able to be determined externally (e.g., in case of a helical scan), but could be derived from the DICOM header of the first medical image or slice arriving at the system 2. Alternatively, it could be hard coded as a scanner of the same make and model usually only provides a specific slice thickness. The following equation shows the calculation, where $N_{slices}$ is the number of slices to be transferred, $L_{scan}$ is the length of the scan path 30 and $d_{slice}$ is the slice thickness:

$$N_{slices} = L_{scan} / d_{slice}.$$

*Linear movement between start and end position*

If the tracking unit 38 and the whole setup (except for the relative movement between patient and imaging device tracker 42) can be considered static, the easiest approach is calculating the Euclidian distance between the imaging device tracker 42 at scan start and scan end position in coordinates of the camera 40. This linear approach might only be a rough approximation but could work in most cases.

*Non-linear movement between start and end position (i.e., if camera is moving as well) without network protocol*

If the tracking unit 38 is capable of supporting machine vision by having an RGB camera onboard, this can also be used to estimate the movement of the CT scanner in space, depending on the spatial movement detected using machine vision. In this case, no tracker 42 would be required. To reduce the influence of a potential movement of the tracking unit 38 during the scan, an acceleration sensor might be used to compensate for such movement, and, if no such sensor is available, machine vison could also be used to find a fixed object in the operating room to which the whole setup can relate. This approach will work best when facing the scanner from the front or back so that it moves in line to the camera of the tracking unit 38. The distance can be estimated from the scanner size difference between scan start and end position. Compared to the tracker measurements explained above, this method may be more error-prone when parts of the scanners are hidden, or the scanner is not seen frontally.

*Time based approach using the network protocol*

As another option, the distance can also be determined based on the scan time. As the speed of the CT scanner might be predictable during a scan (e.g., determined in advance), the scan time will give an estimate of the scan distance, i.e., the length of the scan path 30. This approach might be based on the underlying network protocol only. In other words, the scan time may be derived from one or more messages exchanged between the unit 12 and the system 2, in particular a message from the unit 12 that is transmitted to the system 2 and indicates a scan start confirmation, and another message indicating a scan completion confirmation. In this case, it would not matter whether the trackers 42, 44 are visible to the camera 40 or not. At least for the start position, tracker visibility may be guaranteed as this is typically a precondition for starting a navigated patient scan. However, the navigation system 2 would not need to care whether the CT scanner or imaging device tracker 42 gets invisible during the scan, as all needed information for the actual registration would be already available. For this approach, it may be helpful to know if the scan is axial or helical because depending on that, there may be different scan times for the same scan length.

*Time based approach using tracking to detect scan start and scan end*

When the CT scanner is tracked anyways, the tracking could also be used to detect the movement during the patient scan. When the scanner is seen moving and stopping, the time difference can easily be calculated and hence the distance. For this approach, it may be helpful to know if the scan is axial or helical because depending on that, there may be different scan times for the same length.

*Time based approach using machine vision to detect scan start and scan end*

Alternatively, the time-based approach can also be used with machine vision to detect the CT scanner movement, not considering the position, but just the time it is moving. When the scanner is seen moving and stopping, the time difference can easily be calculated and hence the distance. For this approach, it may be helpful to know if the scan is axial or helical because depending on that, there may be different scan times for the same scan length.

*Differentiating between axial or helical scan*

There are some challenges with the time-based approach. Usually, CT scanners support helical and axial scans where a helical one is quicker as the scanner moved continuously and does not start and stop during the scan. Here again, machine vision or tracking technology might help find out if the scanner moves continuously or stops sometimes during the scan. To detect an axial scanning protocol, it will be enough to see the scanner stopping once during the scan which is more likely than seeing it the whole scan time. Again, machine vision might be helpful as seeing at least parts of the scanner is more often the case than having the tracker trackable.

*Length detection for Axial Scans*

When an axial scan is done, the scanner usually takes a spin to acquire a fixed set of slices. After it moves, it takes another fixed set of slices. This number of slices depends on the make and model of the scanner. In other words, the scanner may acquire a plurality of medical images at each static imaging position 36 along the scan path 30. The number of stops during the axial scan can be converted into the number of expected slices. That number of stops can be taken visually by machine vision or by a tracking of the scanner. It also works if the network protocol contains information about movement stops, hence taking a set of slices.

*Combination of two or more approaches from above*

A single approach from the above-mentioned approaches might not always work, hence, a combination of approaches could be used to determine accurately the length of the scan path 30. Depending on the setup, it might happen that, e.g., the imaging device tracker 42 is not visible to the stereo camera 40 at the end of the scan, but a color camera of the tracking unit 38 can still partly see it.

The described approaches may work best if the slices are transferred individually. If they are transferred as a single file, the approach might be used to estimate the resulting file size. A file compression may then also be considered for determining the total time.

The present technique may help to identify the length of the scan path 30 and therefore the total number of slices to be received by the navigation system 2. The technique may be implemented without changing anything on the CT scanner or the network protocol, by using data the navigation system 2 has available anyway due to established procedures. For the user, it might be an improvement to see the percentage of slices that are already transferred. Transfer progress may provide the user an estimate of how much longer they need to wait for the transfer to complete and start the workflow. This estimation can help users plan their activities accordingly and manage their expectations regarding the completion time of the transfer operation.

It is to be understood that the features described above with reference to the drawings may be combined with the features discussed with reference to the aspects described herein, and vice versa. Also, the example of Fig. 1 shall not limit the method 200 to the depicted exemplary system 100. The method 200 may be performed using a system 100 in which, for example, no trackers 42, 44 are used and/or the image providing unit 12 is configured as an MR scanner or a C-arm scanner. Further modifications may be apparent for those skilled in the art in view of the present disclosure.

## Claims

1. A computer-implemented method (200) for informing a user of a surgical navigation system (2) on a time associated with receiving medical patient scan data from an image providing unit (12), the method being performed by at least one processor (4) of the surgical navigation system (2) and comprising:

   determining (212) a total number of images comprised in medical patient scan data to be received by the surgical navigation system (2) from the image providing unit (12);
   determining (216), based on the total number of images, the total time required to receive, by the surgical navigation system (2), the medical patient scan data from the image providing unit (12); and
   triggering (218) output of an indication of the determined total time or information derived from the determined total time.

2. The method of claim 1, wherein all images comprised in the medical patient scan data are associated with a same medical scan of a same patient.

3. The method of claim 2, wherein the total number of images is determined based on a scan path (30) of the medical scan.

4. The method of claim 3, wherein the scan path (30) defines a relative movement between the patient and an image capturing volume (26) that occurred during the medical scan.

5. The method of claim 3 or 4, wherein the total number of images is determined based on a length of the scan path (30).

6. The method of claims 4 and 5, wherein the length of the scan path (30) is determined based on a spatial start position (32) and a spatial end position (34) of the image capturing volume (26) relative to the patient.

7. The method of any one of claims 4 to 6, wherein the length of the scan path (30) is determined based on a movement time of the image capturing volume (26) relative to the patient and based on a movement speed of the image capturing volume (26) relative to the patient.

8. The method of at least claim 4, wherein the total number of images is determined based on an amount of static, preferably periodical, positions (36) along the scan path (30) between the patient and the image capturing volume (26) that were present when the medical scan was conducted.

**9.** The method of any one of claims 2 to 7, wherein the total number of images is determined based on a type of the medical scan and/or based on a slice thickness of the medical scan.

**10.** The method of any one of claims 3 to 9, wherein at least one of {i} the scan path (30), {ii} the length of the scan path (30), {iii} the spatial start position (32), {iv} the spatial end position (34), {v} the movement time, {vi} the movement speed, {vii} the static positions (36), {viii} the type of the medical scan, and {ix} the slice thickness of the medical scan are captured when the medical scan is conducted.

**11.** The method of claim 10, wherein the capturing is based on a message received (208) from the image providing unit (12) and/or based on tracking data received (204) from a tracking unit (38).

**12.** The method of any one of claims 1 to 11, wherein the total time is determined based on an estimated data transfer rate between the image providing unit (12) and the surgical navigation system (2).

**13.** The method of claim 12, wherein an actual data transfer rate between the image providing unit (12) and the surgical navigation system (2) is determined (220) while receiving the medical patient scan data from the image providing unit (12), and the actual data transfer rate is used to update the determined total time and/or the information derived from the determined total time.

**14.** The method of any one of claims 1 to 13, wherein the information derived from the determined total time is a remaining time required for receiving the medical patient scan data from the image providing unit (12).

**15.** A surgical navigation system (2) comprising at least one processor (4) configured to perform the method according to any one of claims 1 to 14.

**16.** A medical system (100) comprising the surgical navigation system (2) of claim 15 and the image providing unit (12).

**17.** The medical system (100) of claim 16, wherein the image providing unit (12) is connected to the surgical navigation system (2) via a local network connection and/or wherein the image providing unit (12) and the surgical navigation system (2) are configured to be arranged within a same hospital.

**18.** A computer program storing instructions which, when executed by at least one processor (4) of a surgical navigation system (2), cause the at least one processor (4) to perform the method according to any one of claims 1 to 14, wherein, optionally, the computer program is carried by at least one carrier such as a memory (6), a non-transitory computer storage medium or a data stream.

100

10

2

65% complete
120 sec remaining

6

4

8

38

40

40

16

12

14

18

20

42

27

34

36

22

44

30

28, 32

26

24

Fig. 1

200

202 — Establish data connection to tracking unit

204 — Receive tracking data from tracking unit

206 — Establish data connection to image providing unit

208 — Receive message from image providing unit

210 — Determine length of scan path

212 — Determine total number of images comprised in medical patient scan data to be received from image providing unit

214 — Estimate data transfer rate between image providing unit and surgical navigation system

216 — Determine total time required to receive medical patient scan data from image providing unit

218 — Trigger output of indication of total time or information derived from total time

220 — Determine actual data transfer rate between image providing unit and surgical navigation system

Fig. 2

Europäisches
Patentamt
European
Patent Office
Office européen
des brevets

# EUROPEAN SEARCH REPORT

**Application Number**

EP 25 21 2327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2013 079838 A (HITACHI GE NUCLEAR ENERGY LTD) 2 May 2013 (2013-05-02) | 1,2,6,7, 9,11-18 | INV. G16H30/40 |
| Y | * paragraphs [0013], [0023] * ----- | 3-5,8,10 | G16H40/40 |
| Y | US 2021/196219 A1 (JANSEN FLORIBERTUS P HEUKENSFELDT [US] ET AL) 1 July 2021 (2021-07-01) * paragraphs [0040], [0051] * ----- | 3-5,8,10 | |
| A | CN 104 068 885 B (SIEMENS SHANGHAI MED EQUIP LTD) 6 July 2016 (2016-07-06) * the whole document * ----- | 1-18 | |
| A | CN 114 076 913 A (SIEMENS SHENZHEN MAGNETIC RESONANCE LTD) 22 February 2022 (2022-02-22) * the whole document * ----- | 1-18 | |
| A | US 2020/352537 A1 (BAI CHUANYONG [US] ET AL) 12 November 2020 (2020-11-12) * the whole document * ----- | 1-18 | |

**TECHNICAL FIELDS SEARCHED (IPC)**

G16H

The present search report has been drawn up for all claims

| Place of search | Date of completion of the search | Examiner |
|---|---|---|
| Munich | 4 March 2026 | Laub, Christoph |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 21 2327

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2026

| Patent document cited in search report | | | Publication date | Patent family member(s) | | Publication date |
|---|---|---|---|---|---|---|
| JP 2013079838 | A | | 02-05-2013 | NONE | | |
| US 2021196219 | A1 | | 01-07-2021 | CN | 113116370 A | 16-07-2021 |
| | | | | US | 2021196219 A1 | 01-07-2021 |
| CN 104068885 | B | | 06-07-2016 | NONE | | |
| CN 114076913 | A | | 22-02-2022 | CN | 114076913 A | 22-02-2022 |
| | | | | US | 2022057466 A1 | 24-02-2022 |
| US 2020352537 | A1 | | 12-11-2020 | CN | 111566745 A | 21-08-2020 |
| | | | | EP | 3721437 A1 | 14-10-2020 |
| | | | | JP | 7337825 B2 | 04-09-2023 |
| | | | | JP | 2021505907 A | 18-02-2021 |
| | | | | US | 2020352537 A1 | 12-11-2020 |
| | | | | WO | 2019110336 A1 | 13-06-2019 |

EPO FORM P0459

For more details about this annex : see Official Journal of the European Patent Office, No. 12/82